Europäisches Patentamt

European Patent Office

Office européen des brevets

(11) Veröffentlichungsnummer: **0 497 397 A1**

# (12) EUROPÄISCHE PATENTANMELDUNG

(21) Anmeldenummer: **92200084.9**

(22) Anmeldetag: **14.01.92**

(51) Int. Cl.5: **G01N 33/44**, G01N 21/71, B07C 5/342

(30) Priorität: **31.01.91 DE 4102767**

(43) Veröffentlichungstag der Anmeldung:
**05.08.92 Patentblatt 92/32**

(84) Benannte Vertragsstaaten:
**AT BE CH DE DK ES FR GB GR IT LI LU NL SE**

(71) Anmelder: **METALLGESELLSCHAFT AG**
**Reuterweg 14 Postfach 3724**
**W-6000 Frankfurt/M.1(DE)**

(72) Erfinder: **Loree, Thomas R.**
**340 Calle Sierpe**
**Santa Fe, New Mexico 87501(US)**
Erfinder: **Hermes, Robert E.**
**1 Kiowa Lane**
**Los Alamos, New Mexico 87544(US)**

(54) Verfahren zur qualitiven Analyse von Kunststoffteilchen.

(57) Bei einem Verfahren zur qualitativen Analyse von Teilchen verschiedener Kunststoffsorten durch Emissions-Spektralanalyse wird durch Einstrahlung von kurzwelligem Licht ein eine vergleichsweise langwelligere Lichtstrahlung aufweisendes Emissionsspektrum nach Strahlungsintensitäten innerhalb eines relativ engen Wellenlängenbereichs aufgenommen und zur Identifizierung der Kunststoffteilchen herangezogen. Um in großen Mengen anfallende Teilchen unterschiedlicher Kunststoffsorten schnell zu identifizieren und zu sortieren, werden die einzelnen Kunststoffteilchen einem Lichtimpuls ausgesetzt, aus den Strahlungsintensitäten der diskreten Wellenlängen bzw. Wellenlängenbereiche eine Kennzahl nach einem Algorithmus gebildet und durch Vergleich dieser Kennzahl mit vorgegebenen Grenzwerten ein Sortiersignal gewonnen.

EP 0 497 397 A1

Die Erfindung betrifft ein Verfahren zur qualitativen Analyse von Teilchen verschiedener Kunststoffsorten auf der Basis der relativen Konzentration der überwiegend vorkommenden Kunststoffsorte durch Emissions-Spektralanalyse, vorzugsweise Fluoreszenz- oder Raman-Spektralanalyse, bei der das durch Einstrahlung von kurzwelligem Licht, vorzugsweise UV-Licht, erzeugte, eine vergleichsweise langwelligere Lichtstrahlung aufweisende Emissionsspektrum nach Strahlungsintensitäten innerhalb eines relativ engen Wellenlängenbereichs aufgenommen, vorgegebene diskrete Wellenlängen bzw. Wellenlängenbereiche der zu analysierenden Kunststoffe erfaßt und zur Identifizierung der Kunststoffteilchen herangezogen werden.

Der Rückführung von gebrauchten Kunststoffen in den Wirtschafts- und Materialkreislauf kommt gesteigerte Bedeutung zu, um den Erstrohstoff Erdöl und Energie zu sparen und die Müllmenge zu verringern. Denn sowohl der Rohstoff als auch ausreichender Platz für Deponien stehen nur noch begrenzt zur Verfügung. Da es zahlreiche Kunststoffsorten gibt, die sich in ihren definierten mechanisch-technologischen und chemischen Eigenschaften unterscheiden und deshalb auch in unterschiedlichen Bereichen eingesetzt werden, ist es nachteilig, Kunststoffe gemischt zu sammeln und wiederzuverwerten; denn die daraus hergestellten Produkte besitzen nicht nur eine vergleichsweise niedrigere Wertigkeit, sondern sind darüber hinaus auch für eine mehrfache Wiederverwertung nicht geeignet und zum Teil auch untereinander unverträglich. Das bedeutet, jede Kunststoffsorte sollte für die Rückführung in den Materialkreislauf getrennt gesammelt und wiederverarbeitet werden. Diese Forderung ist jedoch nur durch eine eindeutige, gut lesbare Kennzeichnung aller aus Kunststoffen hergestellten Produkte realisierbar. Sobald die Ausführung einer Produktkennzeichnung verbindlich festgelegt ist, sollen die Werkzeugbauer Möglichkeiten entwickeln, die Kennzeichnung in den Werkzeugen zur Herstellung der Kunststoffprodukte einzubringen (Heuel, O.: Eindeutige Produktkennzeichnung als Voraussetzung für ein sortenreines Recycling, Kunststoff-Recycling-Tag, Berlin 21. und 22.09.1989). Ein Mißbrauch ist dadurch jedoch nicht ausgeschlossen. Die Vielzahl der heute verwendeten Kunststoffe von mehr als 100 erschwert das getrennte flächendeckende Sammeln und macht die Einrichtung gesonderter Sammelstellen, die ggf. durch ein System der Abholung bei den Verbrauchern ergänzt werden können, erforderlich, was jedoch in nachteiliger Weise mit einem erheblichen Aufwand verbunden ist.

Bekannt ist die Anwendung der Fluoreszenz-Spektroskopie zur Identifikation von Kunststoff, bei der in die zu untersuchende Substanz kurzwelliges Licht eingestrahlt, das von der Substanz emittierte relativ langwelligere Licht mit Hilfe von geeigneten Spektralapparaten in ein Emissionsspektrum zerlegt, die für die untersuchte Substanz charakteristische Strahlungsintensität in Abhängigkeit von der Wellenlänge erfaßt und gemessen oder in proportionale elektrische Signale umgewandelt und ausgewertet wird (Allen, N.S. and McKellar, J.F.: Chem. and Ind., London 1978, S. 907-913; Firmenschrift: Waterloo Scientific Inc. 1989, Photoluminescence Application Note # 1). Die Anwendung der Emissions-Spektralanalyse auf Kunststoffe ist jedoch bisher im wesentlichen auf die wissenschaftliche Untersuchung von Kunststoffen beschränkt geblieben.

Es ist Aufgabe der vorliegenden Erfindung, das eingangs beschriebene Verfahren zur Emissions-Spektralanalyse so auszugestalten, daß in großen Mengen anfallende Teilchen unterschiedlicher Kunststoffsorten sehr schnell und eindeutig identifizierbar sowie nach Sorten trennbar sind, wobei allerdings der Aufwand der qualitativen Analyse und der Sortierung des einzelnen Kunststoffteilchens deutlich unter der pro Kunststoffteilchen möglichen Wertschöpfung bleibt.

Die Lösung dieser Aufgabe besteht darin, daß jedes Kunststoffteilchen einem kurzwelligen Lichtimpuls ausgesetzt, aus den Strahlungsintensitäten der diskreten Wellenlängen bzw. Wellenlängenbereiche eine Kennzahl nach einem Algorithmus gebildet und durch Vergleich dieser Kennzahl mit vorgegebenen Grenzwerten ein Sortiersignal gewonnen wird.

Im Rahmen der vorzugsweisen Ausgestaltung des Verfahrens wird kurzwelliges Licht mit einer Wellenlänge von 100 bis 500 nm, vorzugsweise 180 bis 400 nm, in die Kunststoffteilchen eingestrahlt.

Die triggerbare Impulsrate des eingestrahlten kurzwelligen Lichts beträgt nach einem weiteren Erfindungsmerkmal 100 bis 600 Impulse/Sekunde, vorzugsweise 100 bis 200 Impulse/Sekunde.

Eine vorzugsweise Ausbildung des Verfahrens ist darin zu sehen, daß die Kunststoffteilchen in einer oder mehreren parallel zueinander verlaufenden Linien vereinzelt mit Abstand hintereinanderliegend in stetigem Fluß von einer Aufgabestelle über einen Meßort, an den sie den kurzwelligen Lichtimpulsen ausgesetzt werden, über einen festgelegten Förderweg zu einer sortenbezogenen Abwurfstelle transportiert werden.

Für den Fall, daß die Kunststoffteilchen verschmutzt sind, kann die Oberfläche durch einen gepulsten Laserstrahl partiell gereinigt werden, wobei der Oberflächenschmutz durch das entstehende Plasma entfernt wird.

Zur Durchführung des vorstehend beschriebenen erfindungsgemäßen Verfahrens ist eine Vorrichtung vorgesehen, die im wesentlichen aus einer UV-Lichtimpulse erzeugenden Lichtquelle, einem Spektralapparat zur Erzeugung eines Emissionsspektrums, einem Spektral-Detektor zur Umwandlung der Strahlungsintensitäten in elektrische Signale und einem Computer zur Verarbeitung der elektrischen Signale für die Erzeugung von Sortiersignalen besteht. Zweckmäßigerweise durchlaufen die einzelnen Kunststoffteilchen beim Einlauf in den Meßort eine Triggereinrichtung, vorzugsweise eine optische Schranke, zur Erzeugung eines Signals, durch das ein Lichtimpuls ausgelöst wird.

Damit es zur Schaltung durch das Ausgangssignal der optischen Schranke kommt, beträgt der Abstand zwischen den einzelnen Kunststoffteilchen wenigstens 1,0 mm. Mit zunehmender Fördergeschwindigkeit wird ein größerer Abstand zwischen den einzelnen Metallteilchen erforderlich.

Die optische Schranke besteht aus einem auf eine Photozelle gerichteten Lichtstrahl, mit dessen Hilfe auch die Länge des einzelnen Kunststoffteilchens gemessen werden kann. Um das Triggersignal zu erzeugen, wird der Lichtstrahl durch das Kunststoffteilchen unterbrochen.

Die optische Schranke kann auch aus einem Laserstrahl oder aus der Kombination von Lichtstrahl und Laserstrahl bestehen, so daß auch ein räumliches Erfassen des einzelnen Kunststoffteilchens möglich ist.

Gegebenenfalls ist eine Einrichtung zur Reinigung der Kunststoffteilchen mittels eines Lasers vorgesehen.

Dem Meßort ist eine Transportstrecke nachgeordnet, entlang derer die Kunststoffteilchen nach ihrer qualitativen Zusammensetzung sortiert werden. Das Sortieren kann auch durch Ausblasen im freien Fall durch die Luft erfolgen.

Eine Vorsortierung der Kunststoffteilchen ist möglich, z.B. nach der Farbe durch die Anwendung von weißem Licht und Absorptions-Spektroskopie.

Als Lichtquelle für die Erzeugung der Lichtimpulse kommt insbesondere ein spektralgefiltertes gepulstes Blitzlicht, ein verschlußgesteuertes gefiltertes Dauerstrich-Blitzlicht, ein Excimerlaser, ein verschlußgesteuerter Dauerstrich-Ionenlaser für den UV-Bereich, ein Impuls-Feststofflaser mit Frequenzvervielfachung für den UV-Bereich, verschlußgesteuertes und gefiltertes Fluoreszenzlicht, verschlußgesteuertes und gefiltertes Sonnenlicht, gefiltertes Licht von Stoßwellen in Edelgasen, gefiltertes Licht von elektrischen Entladungen, verschlußgesteuertes und gefiltertes Licht von kontinuierlichen, elektrischen Lichtbögen, gefiltertes Licht von laserinduzierten Durchschlagsfunken, ein Strobelicht oder photographisches Blitzlicht in Betracht.

Für die Beleuchtungsoptik haben sich insbesondere Kombinationen von lichtdurchlässigen Linsen einschließlich holographischen und Fresnel-Linsen, Kombinationen von konvexen und konkaven, sphärischen und nicht sphärischen Reflektoren, Lichtleiter-Einzelfasern mit Verbindungs- und Ausgangsoptiken zu vorstehend genannten Kombinationen oder Lichtleit-Faserbündel mit Verbindungs- und Ausgangsoptik ggf. mit unterschiedlich ausgebildeten Enden zu vorstehend genannten Kombinationen bewährt.

Als Spektraldiskriminator werden Gitter- und Prisma-Spektrometer, optische Filter aller Art, Interferrometer oder Gitter- und Prisma-Polychromatoren eingesetzt.

Als Detektoren dienen zweckmäßigerweise verstärkte sog. Diodenarrays zur Lichtverstärkung, Diodenarrays, Photovervielfacher, Photodioden, sog. CCD arrays (ladungsgekoppelte Detektorengruppen), Arrays pyroelektrischer Elemente, Videokameras oder spektralselektive Lichtdetektoren, die nur auf vorgegebene Wellenlängen ansprechen.

Die Erfindung ist nachfolgend anhand eines Ausführungsbeispiels in Verbindung mit einer schematischen zeichnerischen Darstellung der Vorrichtung näher erläutert.

Mittels einem mit einer Fördergeschwindigkeit von 3,5 m/s bewegten Gurtbandförderer (1) werden Kunststoffteilchen (2) unterschiedlicher Kunststoffsorten nacheinander dem Meßort (3) zugeführt. Beim Eintritt in den Meßort (3) unterbricht das einzelne Kunststoffteilchen den auf die Photozelle (4) über eine fokussierende Linse (5) sowie über einen Spiegel (6) gelenkten Laserstrahl eines He/Ne-Lasers (7) und wird dadurch erfaßt. Das dabei entstehende Triggersignal wird mittels einer Detektor-Elektronik (8) in ein elektronisches Signal umgewandelt, das zur Auslösung eines UV-Lichtimpulses durch die Blitzlichtlampe (9) benutzt wird. Das durch den Filter (10) gefilterte UV-Licht wird mit Hilfe der Beleuchtungsoptik (11) auf die Oberfläche des Kunststoffteilchens (2) fokussiert. Die von dem Kunststoffteilchen (2) emittierte Strahlung wird über eine Relaisoptik (12) von einer Spektraleinrichtung (13) mit nachgeschaltetem Detektor (14) aufgenommen. Aus den aus dem Detektor (14) austretenden elektronischen Signalen werden durch den Computer (15) zahlenmäßige Verhältniswerte gebildet, die mit vorgegebenen Grenzwerten verglichen und dann Signale für die Ansteuerung einer Sortiereinrichtung gewonnen werden.

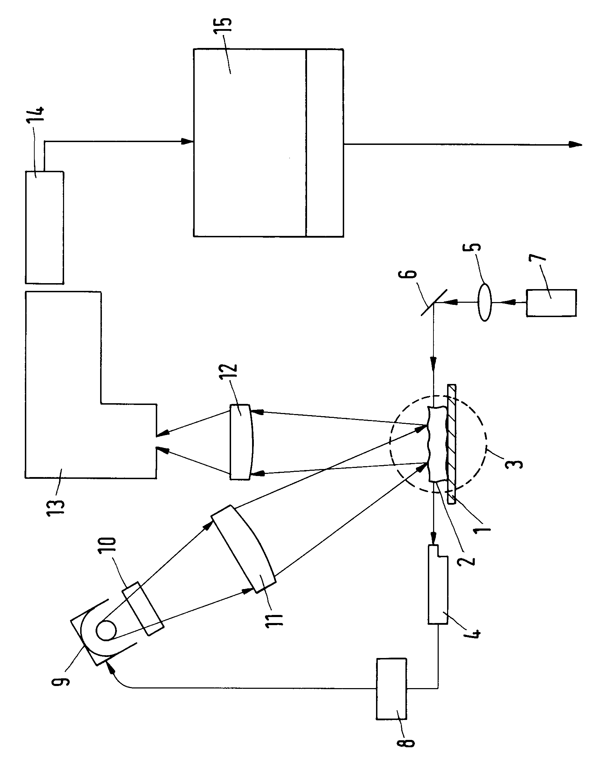

**Patentansprüche**

1. Verfahren zur qualitativen Analyse von Teilchen verschiedener Kunststoffsorten auf der Basis der relativen Konzentration der uberwiegend vorkommenden Kunststoffsorte durch Emissions-Spektralanalyse, vorzugsweise Fluoreszenz- oder Raman-Spektralanalyse, bei der das durch Einstrahlung von kurzwelligem Licht, vorzugsweise UV-Licht, erzeugte, eine vergleichsweise langwelligere Lichtstrahlung aufweisende Emissionsspektrum nach Strahlungsintensitäten innerhalb eines relativ engen Wellenlängenbereichs aufgenommen, vorgegebene diskrete Wellenlängen bzw. Wellenlängenbereiche der zu analysierenden Kunststoffe erfaßt und zur Identifizierung der Kunststoffteilchen herangezogen werden, dadurch gekennzeichnet, daß die einzelnen Kunststoffteilchen einem Lichtimpuls ausgesetzt, aus den Strahlungsintensitäten der diskreten Wellenlängen bzw. Wellenlängenbereiche eine Kennzahl nach einem Algorithmus gebildet und durch Vergleich dieser Kennzahl mit vorgegebenen Grenzwerten ein Sortiersignal gewonnen wird.

2. Verfahren nach Anspruch 1, dadurch gekennzeichnet, daß kurzwelliges Licht mit einer Wellenlänge von 100 bis 500 nm, vorzugsweise 180 bis 400 nm, in die Kunststoffteilchen eingebracht wird.

3. Verfahren nach den Ansprüchen 1 und 2, dadurch gekennzeichnet, daß die triggerbare Impulsrate des eingestrahlten kurzwelligen Lichts 100 bis 600 Impulse/Sekunde, vorzugsweise 100 bis 200 Impulse/Sekunde, beträgt.

4. Verfahren nach den Ansprüchen 1 bis 3, dadurch gekennzeichnet, daß Kunststoffteilchen in einer oder mehreren parallel zueinander verlaufenden Linien vereinzelt mit Abstand hintereinanderliegend in stetigem Fluß von einer Aufgabestelle über einen Meßort, an dem sie den kurzwelligen Lichtimpulsen ausgesetzt werden, über einen festgelegten Förderweg zu einer sortenbezogenen Abwurfstelle transportiert werden.

5. Verfahren nach Anspruch 4, dadurch gekennzeichnet, daß der Abstand zwischen den einzelnen Kunststoffteilchen wenigstens 1,0 mm beträgt.

6. Verfahren nach den Ansprüchen 1 bis 5, dadurch gekennzeichnet, daß die Oberfläche der verschmutzten Kunststoffteilchen durch einen gepulsten Laserstrahl partiell gereinigt wird.

7. Verfahren nach einem der vorhergehenden Ansprüche, dadurch gekennzeichnet, daß die Kunststoffteilchen vorsortiert werden, beispielsweise nach Farbe durch Bestrahlung mit weißem Licht und unter Anwendung der Absorptions-Spektroskopie.

8. Vorrichtung zur Durchführung des Verfahrens nach den Ansprüchen 1 bis 7, gekennzeichnet durch

a) eine kurzwellige Lichtimpulse erzeugende Lichtquelle (9),

b) einen Spektralapparat (13) zur Erzeugung eines Emissionsspektrums,

c) einen Detektor (14) zur Umwandlung der Strahlungsintensitäten in elektrische Signale,

d) einen Computer (15) zur Verarbeitung der elektrischen Signale zur Erzeugung von Sortiersignalen.

9. Vorrichtung nach Anspruch 8, gekennzeichnet durch eine Triggereinrichtung zur Erzeugung eines Signals für die Auslösung eines Lichtimpulses.

10. Vorrichtung nach den Ansprüchen 8 und 9, dadurch gekennzeichnet, daß die Triggereinrichtung aus einem auf eine Photozelle (4) gerichteten Lichtstrahl besteht.

11. Vorrichtung nach den Ansprüchen 8 bis 10, dadurch gekennzeichnet, daß die Triggereinrichtung aus einem Laser gebildet ist.

12. Vorrichtung nach den Ansprüchen 8 bis 11, gekennzeichnet durch eine Einrichtung zur partiellen Reinigung der Oberfläche des Kunststoffteilchens mittels eines Lasers.

13. Vorrichtung nach den Ansprüchen 8 bis 12, dadurch gekennzeichnet, daß dem Meßort (3) eine Transportvorrichtung nachgeordnet ist.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| Y | EP-A-0 293 983 (METALLGESELLSCHAFT AG) <br> * das ganze Dokument * <br> --- | 1-13 | G01N33/44 <br> G01N21/71 <br> B07C5/342 |
| Y | EP-A-0 341 096 (GERSAN ESTABLISHMENT) <br> * Spalte 1, Zeile 10 - Zeile 14 * <br> * Spalte 2, Zeile 5 - Zeile 11 * <br> * Zusammenfassung * <br> --- | 1-13 | |
| A,D | CHEMISTRY AND INDUSTRY <br> 2. Dezember 1978, LONDON <br> Seiten 907 - 913; <br> N. S. ALLEN ET AL.: 'Luminescence applications in commercial polymers' <br> * das ganze Dokument * <br> --- | 1 | |
| A | GB-A-2 234 347 (BXL PLASTICS LTD.) <br> * Zusammenfassung * <br> --- | 1 | |
| A,P | DE-A-4 004 627 (FRIED. KRUPP GMBH) <br> * das ganze Dokument * <br> --- | 1,8 | |
| A | EP-A-0 068 086 (CHEMISCHE WERKE HÜLS AG) <br> * Zusammenfassung * <br> ----- | 1 | **RECHERCHIERTE SACHGEBIETE (Int. Cl.5 )** <br><br> G01N <br> B07C |

Der vorliegende Recherchenbericht wurde für alle Patentansprüche erstellt

| Recherchenort | Abschlußdatum der Recherche | Prüfer |
|---|---|---|
| BERLIN | 13 MAI 1992 | BRISON O.P. |